# EUROPEAN PATENT SPECIFICATION

(11) **EP 0 842 426 B1**
(45) Date of publication and mention of the grant of the patent: **04.09.2002**
(21) Application number: 96926484.5
(22) Date of filing: 05.08.1996
(51) Int. Cl.: G01N 33/50, G01N 33/53

(54) **IMPROVEMENTS IN OR RELATING TO METHODS OF SCREENING SUBSTANCES**
VERBESSERUNGEN IN ZUSAMMENHANG MIT SCREENINGVERFAHREN
PERFECTIONNEMENTS RELATIFS A LA SELECTION DE SUBSTANCES

(30) Priority: 05.08.1995 GB 9516138
(43) Date of publication of application: 20.05.1998
(73) Proprietor: Biofocus Discovery Limited, Cambridge CB4 4FD (GB)
(72) Inventor: RUSSELL, Stephen, James, Cambridge CB2 5ER (GB); MORLING, Frances, Joanne, Cambridge CB1 4TH (GB); PENG, Kah-Whye, Cambridge CB1 2ET (GB)
(74) Representative: Lipscombe, Martin John
(86) International application number: GB9601905
(87) International publication number: WO97006435

(56) References cited:
- WO-A-93/00103
- WO-A-94/06920
- WO-A-94/27643
- WO-A-96/00294
- NATURE, vol. 352, 15 August 1991, pages 624-628, XP002019761 T. CLACKSON ET AL.: "Making antibody fragments using phage display libraries."
- NUCLEIC ACIDS RESEARCH, vol. 21, no. 5, 1993, pages 1081-1085, XP002019762 S. J. RUSSELL ET AL.: "Retroviral vectors displaying functional antibody fragments."
- PROTEIN ENGINEERING, vol. 2, no. 1, 1988, pages 63-68, XP002019763 P. M. LEHTOVAARA ET AL.: "A new method for random mutagenesis of complete genes: enzymatic generation of mutant libraries in vitro." cited in the application

### SEQUENCE LISTING

(1) GENERAL INFORMATION:
   (i) APPLICANT:
      (A) NAME: Medical Research Council
      (B) STREET: 20 Park Crescent
      (C) CITY: London
      (E) COUNTRY: United Kingdom
      (F) POSTAL CODE (ZIP): W1N 4AL
      (G) TELEPHONE: (0171) 636 5422
      (H) TELEFAX: (0171) 323 1331
   (ii) TITLE OF INVENTION: Improvements in or Relating to Methods of Screening Substances
   (iii) NUMBER OF SEQUENCES: 5
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (xi) SEQUENCE DESCRIPTION: SEQ ID NO: 1:
   ATGCTCAGAG GGGTCAGTAC GGCCCAGCCG GCCATGGCCA ATAGTGACTC TGAATGTCCC 60
(2) INFORMATION FOR SEQ ID NO: 2:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (xi) SEQUENCE DESCRIPTION: SEQ ID NO: 2:
   ACCTGAAGTG GTGGGAACTG CGCGCGGCCG CATGTGGGGG TCCAGACTCC 50
(2) INFORMATION FOR SEQ ID NO: 3:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 3:
   TCTAGACTGA CATGGCGCGT 20
(2) INFORMATION FOR SEQ ID NO: 4:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (xi) SEQUENCE DESCRIPTION: SEQ ID NO: 4:
   GGTGACTCTC CAGGTTAC 18
(2) INFORMATION FOR SEQ ID NO: 5:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO: 5:
   GTAAGGTCAG GCCACCAGGT                                        20

## Description

### Field of the Invention

This invention relates to a method of screening substances for reduced ability to bind to a specified target molecule.

### Background of the Invention

### a) Genetic display library selection

The isolation of genes encoding proteins with known binding properties has recently been facilitated by selection technologies utilising genetic display packages. The genes encoding the protein are packaged such that the encoded protein is displayed on the outside of the package. The package is then selected by its binding affinity (for example an encoded antigen by binding to solid phase antibody), and replicated. The tight linkage between genes and encoded protein allow packages to be selected in rounds of binding and amplification, leading to selection factors of more than one in a million. Replicable packages have included mammalian cells - used to isolate the genes encoding lymphocyte surface markers from cDNA libraries (Strengelin *et al*, 1988 EMBO J 7 p1053-1059), and filamentous bacteriophage - used to isolate the genes encoding antibody fragments and other proteins (McCafferty *et al*, 1990 Nature 348 p552-554; Bass *et al*, 1990 Proteins: Struct. Funct. Genet. 8 p309-314; Marks *et al*, 1991 J. Mol. Biol. 222 p581-597; Hoogenboom & Winter, 1992 J. Mol. Biol. 227 p381-388). More recently, recombinant retroviruses have been used as replicable display packages, constructs suitable for the generation of retroviral display libraries have been disclosed, and the selection of retroviral display libraries has been proposed to facilitate the isolation of genes encoding ligands that can target retroviral entry into mammalian cells through novel cell surface receptors (WO94/06920. Medical Research Council).

There are several important differences between retroviral display and phage display.

Most significantly, retroviruses can infect mammalian cells whereas phage cannot. In addition, phage display relies on bacterial expression of the displayed polypeptide ligand which is often problematic since polypeptide ligands originating from mammalian cells frequently fail to fold correctly or function normally when expressed in bacteria. This deficiency stems largely from the lack of a bacterial compartment equivalent to the endoplasmic reticulum of mammalian cells which is equipped with enzyme systems for post-translational modification (e.g. glycosyl transferases) and folding (e.g. chaperonins, disulphide and proline isomerases) of polypeptide ligands, as well as the sorting and expulsion of misfolded variants.

Thus, packages displaying ligands with high binding affinity for a specified target antigen or receptor are readily selected from diverse libraries of genetic display packages. However, the converse is not true. There are no reliable methods established for the selection of packages displaying ligands with reduced ability to bind to a specified target.

### b) Drug discovery

Cells use their surface receptors to monitor and respond to subtle changes in the composition of their immediate environment. Drugs which block or activate specific receptors can therefore be used to modify cellular functions for medicinal purposes. During the last two or three decades some of the most profitable drugs have been compounds that block ligand binding or directly stimulate cell surface receptors. Molecular biology has now uncovered a plethora of high molecular weight polypeptide ligands (for examples, see WO 94/27643 [Targeted Genetics Corporation] and references therein) with diverse biological activities and the Human Genome Project promises to uncover many more. Thus, a major challenge for the pharmaceutical industry is to discover new drugs that block or mimic the effects of these ligands, or exhibit greater specificity for receptor subtypes (Luyten & Leysen 1993 Trends in Biotechnology 11 p247).

Searching the "chemical space" around a natural ligand by screening synthetic analogues has been a very successful route to the discovery of new drugs in the past. The evolution of adrenergic receptor drug specificity is a case in point. Structural information about the ligand, the receptor and the ligand-receptor complex is therefore of great value in drug discovery since it can point to classes of compounds that are more likely to yield active leads in the screening process. Good structural information can also provide the rationale for *de novo* synthesis of new classes of chemical compounds that are likely to bind to a given ligand or receptor (rational drug design). Unfortunately, few ligands, receptors or ligand-receptor complexes have yielded to direct structural analysis and computer models of these proteins are often suspect, built as they are on the basis of known structures of homologous proteins and scanty site-directed mutagenesis data. Comprehensive data on the effects that individual point mutations have on the binding characteristics of a ligand can be invaluable for model refinement. However, such information is hard to obtain since it takes considerable time and effort to construct, express, purify and characterise each point mutated polypeptide.

There is therefore a need for rapid (preferably library-based) strategies to glean information on multiple point mutations in a short time period. If a polypeptide ligand happens to be expressible in bacterial cells (and this is often not the case), identification of mutations that confer enhanced affinity for a receptor can be accomplished using phage display libraries. However, there is currently no system that allows the rapid selection of correctly folded mutants of a polypeptide ligand which exhibit reduced affinity for their receptor.

### c) Ligand-dependent, receptor-mediated retroviral sequestration

Retroviral envelope glycoproteins mediate specific viral attachment to cell surface receptors and subsequently trigger fusion between the viral envelope and the target cell membrane. Retroviral envelope glycoproteins consist of an external glycoprotein moiety (SU) noncovalently attached at its C-terminus to a smaller transmembrane polypeptide moiety (TM). Each surface projection (or spike), visible by electron microscopy on the viral surface is a trimer of identical envelope glycoprotein subunits. SU comprises two domains connected by a proline-rich hinge, the N-terminal domain conferring receptor specificity and exhibiting a high degree of conservation between Murine Leukaemia Viruses (MLVs, which are generally typical retroviruses) with different host ranges (Battini *et al*, 1992 J. Virol. 66 p1468-1475).

There has previously been disclosed a general method that allows the display of a (glyco)polypeptide ligand on the surface of a retroviral vector as a genetically encoded extension of the viral envelope protein (WO94/06920, Medical Research Council). The engineered retroviral vector then adopts the binding specificity of the displayed ligand. To date we have displayed many different polypeptide ligands on murine leukaemia virus - based retroviral vectors, including single chain antibodies, cellular growth factors and immunoglobulin binding domains (WO94/06920, Medical Research Council; WO 96/00294, Medical Research Council; Cosset *et al* 1994 Gene Therapy 1 pS1; Nilson *et al* 1994 Gene Therapy 1 pS17). In principle, our technology should allow the display of many different structural classes of binding domains on retroviral vectors, including glycopolypeptides and glycoproteins.

We also recently discovered a novel biological phenomenon called ligand-dependent, receptor-mediated viral sequestration (illustrated in example 1 below). As an example by way of explanation, a polypeptide ligand may be fused (by genetic engineering) to the envelope protein of an MLV-based retroviral vector such that the envelope protein to which it has been grafted remains substantially intact and capable of binding to its natural receptor, and the fused nonviral polypeptide ligand is displayed on the viral surface. The virus displaying the fused non-viral polypeptide ligand is then capable of multivalent attachment to the natural virus receptor and to the cognate receptor for the non-viral ligand; attachment to the natural virus receptor leads to infection of the target cell, whereas attachment to the cellular receptor for the displayed non-viral ligand may not lead to infection of the target cell. Where the target cell expresses both species of receptor and attachment through the displayed non-viral ligand does not lead to infection, the two binding reactions (envelope protein to natural receptor and non-viral ligand to its cognate receptor) proceed in competition and the infectivity of the virus for the target cells is reduced in proportion to the efficiency with which the second binding reaction competes virus away from the natural virus receptor.

For example, when epidermal growth factor (EGF) was displayed on an amphotropic retroviral vector, the engineered vector bound preferentially to EGF receptors present on EGF receptor-positive human cells and gene transfer did not occur. EGF receptor-negative cells were fully susceptible to the engineered retroviral vector but showed reduced susceptibility when they were genetically modified to express EGF receptors. The reduction in susceptibility was in proportion to the level of EGF receptor expression. Moreover, when soluble EGF was added to competitively inhibit virus capture by the EGF receptors, gene transfer was restored (see WO 96/00294). The loss of infectivity of the EGF-displaying virus on EGF receptor expressing cells is believed to reflect a block to membrane fusion between the viral envelope and the target cell plasma membrane.

The degree to which gene transfer is inhibited depends on the relative affinities of the two binding reactions (envelope protein to natural receptor and non-viral ligand to its cognate receptor), the relative densities of the two receptors on the target cell surface, and the relative densities of the non-viral ligand and the intact envelope protein on the viral surface. Inhibition of gene transfer is additionally influenced by intrinsic properties of the receptor for the non-viral ligand, such as the distance it projects from the target cell membrane, its mobility within the target cell membrane and its half life on the cell surface after engagement of ligand.

### Summary of the Invention

In a first aspect the invention provides a method of screening a plurality of altered first members of a specific binding pair ("sbp") for reduced ability to bind to the second member of the sbp compared to an unaltered first member; the method comprising introducing a plurality of nucleic acid sequences, each encoding a respective altered first member of the sbp, into a plurality of viral display packages so as to form a viral display package library in which each viral display package displays on its surface an altered first member of the sbp encoded by a respective nucleic acid sequence comprised within the viral display package and an infection-competent binding moiety capable of mediating infection of a susceptible target cell; contacting the viral display packages with a plurality of target cells which express on their surface a receptor molecule for the infection-competent binding moiety, such that binding to the receptor molecule tends to facilitate infection of a target cell by the viral display package, said target cells also expressing on their surface the second member of the sbp, binding to which, via the displayed altered first member of the sbp, tends to inhibit infection of a target cell by the viral display package, such that those viral display packages displaying altered first members with reduced ability to bind to the second member of the sbp will tend to infect target cells more successfully; and recovering from infected target cells or their progeny nucleic acid comprising those nucleic acid sequences which encode altered first members with reduced ability to bind to the second member of the sbp.

The viral display package could be based on filamentous bacteriophage or, more preferably, any virus capable of infecting a eukaryotic cell, such as a retrovirus (in particular, a C type retrovirus, preferably MLV). Other suitable viruses may include Vesicular Stomatitis virus (VSV), influenza virus, Semliki Forest virus, Sindbis virus, Sendai virus or Adenoviruses. Viral display packages capable of infecting eukaryotic cells are found particularly useful as they allow for the use of eukaryotic target cells, which are more likely to express the second member of the sbp in an appropriate conformation than are prokaryotic target cells, for the reasons outlined above. In particular, the target cells are desirably mammalian (preferably rat, mouse or human cells, conveniently a cell line) which allows for more accurate extrapolation of the results of the screening method to a potential clinical or veterinary situation.

Conveniently the first member and/or the second member of the sbp will be a polypeptide (with or without glycosylation, as appropriate). Advantageously the target cell will naturally express the receptor molecule and the second member of the sbp, although it will be apparent to those skilled in the art that the sequence directing for the expression of one or both of these molecules may be articifically introduced into the target cell and that this may be done, even where both molecules are naturally expressed in the target cell, in order to increase the level of expression of one or both thereof.

Preferably the altered first member of the sbp will be displayed on the viral display package as part of a fusion polypeptide, advantageously the altered first member may be fused with the infection-competent binding moiety. Conveniently the fusion will be with an effective portion of a viral envelope protein or glycoprotein (by "effective portion" is meant a portion of the viral envelope protein sufficient substantially to retain its natural binding affinity). Desirably the envelope protein will be substantially intact. In a preferred embodiment the altered first member of the sbp is displayed as an N-terminal fusion with a viral envelope glycoprotein (the phrase "N-terminal", as used herein, is intended to apply to fusions at the N-terminal region of the envelope glycoprotein [i.e. within 30 or so amino acids of the N-terminal of the molecule, preferably within 20 amino acids, and more preferably within 10 amino acids] and not solely to fusions at the extreme N-terminal of the molecule, although the inventors have found that fusions nearest the N-terminal are generally optimal in terms of preserving the infection-competence of the retroviral envelope protein with, for example, fusions at amino acid residue 1 being preferred to fusions between residues 6 and 7 of the retroviral envelope protein.

In a particular embodiment, the altered first member of the sbp is displayed as a fusion with the 4070A MLV envelope glycoprotein.

The viral display package will desirably comprise a transferrable label which is transferred to, or combines with, the target cell upon infection by the viral display package. The transferable label is preferably a selectable marker, such as an antibiotic-resistance gene or the like, which facilitates identification and selection of target cells which have been infected by a viral display package.

There is evidence to suggest that if the altered first member of the sbp does not fold in an appropriate manner, it will not become incorporated in the viral display package. The method of the invention thus provides a means of rapidly screening a large number of altered molecules which are correctly folded and identifying those which, nevertheless, exhibit reduced binding ability compared to the unaltered molecule. Such correctly folded "non-binding" substances may have reduced binding affinity and/or reduced binding avidity. Preferably the method of the invention will be arranged such that substances may be detected which have lost substantially all affinity for the second member of the sbp. The method of the invention has considerable potential use in the search for new drugs.

Typically the nucleic acid sequences encoding the altered first member of the sbp will be prepared by random mutagenesis of the sequence coding for the unaltered first member. A number of methods of random mutagenesis are known to those skilled in the art (e.g. Matteuci & Heyneker 1983 Nucl. Acids Res. 11, 3113; Wells *et al.*, 1985 Gene 34, 315; Nerr *et al.*, 1988 DNA 7, 127; Kadonaga & Knowles 1985 Nucl. Acids Res. 13, 1733; Meyers *et al.*, 1985 Science 229, 242; and Lehtovaara *et al.*, 1988 Protein Eng. 2, 63). Conveniently, the nucleic acid sequences coding for the altered first member will be at least 90% homologous with the sequence coding for the wild type unaltered first member.

Thus, for example, the plurality of altered first members may all be altered (relative to the unaltered sequence) at a single amino acid residue, or may be altered at a number of different amino acid residues (typically up to 10% of residues may be altered). The residues selected for alteration may be random or may be targeted on the basis of a prior knowledge of the structure of the first and/or second member of the binding pair. The alterations will typically take the form of amino acid substitutions, but may also comprise small (e.g. 1-10 amino acids) deletions or additions.

Typically the method of the invention will also be performed using an unaltered first member of the binding pair included as a control, against which the activity of the variant altered first members may be directly compared.

In another aspect the invention provides a kit for performing a method of screening according to the invention defined above, said kit comprising a nucleic acid construct suitable for accepting a sequence encoding a first member of an sbp; retroviral packaging cells for producing viral display packages incorporating the nucleic acid construct; target cells expressing on their surface a receptor which allows infection by the viral display packages and also expressing the second member of the sbp and instructions for performing the method of the invention. Preferably the kit will further comprise one or more of the following components: means for introducing mutations in the nucleic acid encoding the first member of the sbp; and detection means for detecting infection of a target cell by a viral display package.

In particular we disclose a novel method in which a retroviral display library is used for the rapid identification of mutations that confer a folded "nonbinding" phenotype on a polypeptide first member of an sbp (hereinafter occasionally referred to as the "ligand") that is known to cause receptor-mediated retroviral sequestration. The method is exemplified below.

In a first step, the nucleic acid sequence coding for the ligand is randomised (using any one of a number of methods known to those who are skilled in the art) to create a library of sequences which, typically, are more than 90 percent homologous to the original nucleic acid sequence. The library of nucleic acid sequences is then ligated into an appropriately designed retroviral envelope expression plasmid and the resulting plasmid library is transfected into appropriate complementing cells (e.g. mammalian cells expressing MLV gag and pol proteins) to generate a retrovirus display library. Each species in the retrovirus display library displays the ligand or a variant thereof (i.e. in which the amino acid sequence has been altered) on its surface, and encapsidates the corresponding (mutated or unmutated) nucleic acid sequence. It is assumed that correct folding and transport of each chimaeric envelope protein (displaying a mutated ligand) to the surface of the complementing (virus-producing) cells will be required for its incorporation into retroviral particles - mutations that interfere with ligand folding will therefore be effectively excluded from the library.

The library is then placed in contact with receptor-positive mammalian target cells that sequester retroviruses displaying the unmutated ligand - the retroviral nucleic acid is successfully transferred to the target cells only by members of the library whose mutated polypeptide domain shows reduced affinity for the receptor. The transferred nucleic acid sequences are then recovered from the chromosomal DNA of the target cells (e.g. by PCR amplification), and sequenced to identify the mutations that conferred the folded nonbinding phenotype .

A typical DNA construct suitable for the generation and selection of a library of such genetic display packages should be small enough to allow convenient manipulation and should comprise:
(1) A eukaryotic cell expression cassette whose encoded polypeptide comprises a structural signal to direct its efficient incorporation into viral particles and a nonviral moiety (peptide or polypeptide) which is displayed at the surface of the virion. The polypeptide should include a displayed viral moiety capable of binding to receptors present on the chosen target cells so as to mediate infection therof.
(2) Unique, non-complementary restriction sites flanking the sequences which encode the nonviral moiety to facilitate the directional cloning of a diverse library of peptide or polypeptide sequences into this site.
(3) A viral encapsidation signal sequence to direct the incorporation of the sequences encoding the displayed polypeptide into the viral particles before they leave the packaging cell.
(4) Cis-acting sequences which, in the presence of appropriate trans-acting factors, mediate amplification of the copy number of the nucleic acid sequences which code for the displayed polypeptide and associated encapsidation signal sequence.
(5) A bacterial plasmid origin of replication and antibiotic resistance marker gene to facilitate amplification of the plasmid in a bacterial host strain (e.g. *Escherichia coli*).

In the case of a retroviral genetic display package, long terminal repeat sequences, a tRNA primer binding site and a polypurine tract are preferably included to ensure reverse transcription and integration of the encapsidated RNA in an infected target cell. It may also be desirable to include a selectable marker gene in the encapsidated nucleic acid to facilitate recovery of sequences encoding the displayed polypeptide from infected target cells after a round of selection. A plasmid (pNIPenv) that exhibits all of the features necessary for the generation of retroviral genetic display libraries was disclosed in patent application WO94/06920 (Medical Research Council).

Using similar cloning strategies to that employed to generate pNIPenv, unique *Sfi*I and *Not*I restriction sites have now been inserted between the codons for the 6th and 7th amino acids and immediately 5' of the codon for the first amino acid of the mature Moloney MLV envelope glycoprotein in a full-length infectious molecular clone of MoMLV (Shoemaker *et al*, 1980 Proc. Natl. Acad. Sci. USA 77 p3932-3936), without disruption of the translational reading frame. The *Sfi*I site in the gag coding region of this vector (Shinnick *et al*, 1981 Nature 293 p543-548) has been removed so that the vector can be used to generate libraries of replication-competent retroviruses displaying variegated peptides, polypeptides or glycopolypeptides. We have also generated similar constructs suitable for the generation of retrovirus display libraries in which the displayed polypeptides are expressed as N-terminal extensions of the amphotropic 4070A MLV envelope glycoprotein.

A logical extension of this approach to the identification of mutations that reduce binding affinity without disrupting protein folding is the construction of vectors of similar design for the encapsidation of nucleic acid encoding a displayed nonviral polypeptide on any virus of any family of viruses which can infect eukaryotic cells. The construction and use of similar vectors based on other virus families is particularly convenient for enveloped viruses of limited size and complexity such as Vesicular Stomatitis Virus, Influenza Virus, Semliki Forest Virus, Sindibis Virus and Sendai Virus which display oligomeric membrane spike glycoproteins. The construction and use of similar vectors derived from viruses of the Adenovirus family may also be advantageous since the adenovirus fibre glycoprotein has important features in common with the MoMLV envelope glycoprotein in that it is a glycosylated homotrimer (Mullis *et al*, 1990 J. Virol. 64 p5317-5323; Devaux *et al*, 1990 J. Mol. Biol. 215 p567-588) which is visible by electron microscopy as a spike projecting from the surface of the virion (Ruigrok *et al*, 1990 J. Mol. Biol. 215 p589-596).

The encapsidated nucleic acid need not encode the displayed peptide, polypeptide or glycopolypeptide as a fusion with a viral spike glycoprotein, although this is preferred. We also envisage the use of viruses in which the encapsidated nucleic acid encodes an engineered derivative of a nonviral protein such as CD4 which may be efficiently incorporated into the particles and which has been engineered to display a nonviral peptide, polypeptide or glycopolypeptide. We also envisage the use of viruses in which the nonviral peptide, polypeptide or glycopolypeptide is anchored in the virus particle by fusion to a synthetic polypeptide.

The generation of a diverse library of recombinant retroviruses has been demonstrated previously by transient transfection of retroviral plasmids into retroviral packaging cells (Murphy & Efstratiadis, 1987 Proc. Natl. Acad. Sci. USA 84 p8277-8281). The library size is limited by the efficiency of plasmid transfection, first into *E. coli* for growth and purification of the plasmid ligation product and second into the retroviral packaging cells for generation of the retroviral genetic display library. Employing recently developed and highly efficient methods of gene delivery to mammalian cells (Curiel *et al*, 1992 Hum. Gene Ther. 3 p147-154), we estimate that a library size of 10⁸ retroviral genetic display packages should be possible.

The experimental parameters which are amenable to manipulation and which may affect the outcome of the selection procedure include:

### 1. The underlying composition of the viruses in the library, (which is in turn determined by the design of the vector plasmid and packaging system used to generate the library).

The nucleic acid encapsidated in the viruses may be non-defective (i.e. competent for the production of infectious progeny viruses in infected target cells) or defective and may preferably (but not essentially) include selectable marker genes (conferring antibiotic resistance, for example).

The surface composition of the viruses in the library (and hence their starting host range properties) is determined initially by the choice of the packaging system. For example, by appropriate choice of packaging cells, the RNA transcript and encoded fusion protein of a retrovirus display library can be incorporated into retroviruses which also display unmodified mouse ecotropic MLV envelope proteins, unmodified mouse amphotropic MLV envelope proteins, or which do not display unmodified envelope proteins.

The starting host range of the viruses in the library may be further determined by the nature of the viral moieties of the modified spike glycoproteins, employed to anchor the displayed non-viral polypeptides in the viruses (mouse ecotropic or amphotropic, for example).

### 2. The composition of the library of displayed polypeptides

This may comprise a diverse collection of variants of a peptide, antibody fragment (Fv, scFv, Fab), T-cell receptor, growth factor, cytokine, viral protein or enzyme generated by random or targeted mutagenesis.

The size of the library (i.e. diversity, number of viruses displaying a unique nonviral polypeptide) is determined by the methods used to generate diversity in the vector inserts encoding the displayed polypeptides, the efficiency of their introduction into the vector cloning site, the efficiency of the introduction of the vector into *E. coli* and the efficiency of introduction of the vector plasmid library into the packaging cells. There are numerous methods available to generate diversity in a library of viruses, and these have been described previously in relation to phage antibody libraries. The lox-cre system, or other recombinase systems which are active in eukaryotic cells, may be employed to further increase the diversity of the library by the random recombination of components of the vector inserts.

The final virus titre in the library will be a product of the diversity of the library and the number of copies of each species in the library. Titre is a function of the efficiency of the packaging system, which relates to the efficiency of expression of the vector and helper functions (i.e. viral structural and nonstructural proteins), and to the efficiency of amplification and encapsidation of the vector genome in the packaging cells. The virus titre could therefore be enhanced, for example, by the use of high copy number episomally replicating plasmids and/or strong cellular promoter/enhancers and by the appropriate selection of highly efficient encapsidation signal sequences.

### 3. The composition of the target cells

The species of origin, tissue of origin, state of differentiation, state of activation, state of synchrony and proliferative status of the target cells are important variables which will influence the outcome of the selection strategy.

The degree to which gene transfer is inhibited depends on the relative affinities of the two binding reactions (envelope protein to natural receptor and ligand [first member of the sbp] to its cognate receptor [second member of the sbp]) and on the absolute and relative densities of the two receptors on the target cell surface. The ligand is typically of non-viral origin. Inhibition of gene transfer is additionally influenced by intrinsic properties of the receptor for the (non-viral) ligand, such as the distance it projects from the target cell membrane, its mobility within the target cell membrane and its half life on the cell surface after engagement of ligand. The absolute number and purity of the eukaryotic target cells can also be varied.

### 4. The conditions under which the library of viral display packages is contacted with the target cells.

The time, temperature, pH, composition of medium, presence of competitor antigen or blocking proteins etc may be varied to influence the outcome of the selection procedure. The target cells may alternatively be contacted with a library of virus-producing cells (commonly known as "packaging cells"), rather than directly with viral display packages.

### 5. The treatment of the target cells subsequent to their exposure to the virus library

The target cells may be maintained in tissue culture (or in a living organism) for a variable period of time without further selection. A pure population of target cells may be selected from a mixed population of target cells, for example by fluorescent staining and fluorescence-activated cell sorting. The target cells may be stimulated, for example by the application of selected growth factors or cytokines.

The target cells may selected on the basis of their expression of the delivered viral nucleic acid. If the viral nucleic acid encodes an antibiotic resistance marker such as neomycin phosphotransferase, exposure of the cells to the antibiotic G418 will select for those cells expressing the viral nucleic acid. The viral nucleic acid sequences can be recovered from the infected target cell population before or after it has been subjected to a further selection process to eliminate those cells which were not successfully infected or which do not express the protein(s) encoded by the delivered nucleic acid. Alternatively, the target cell population could be stained with fluorescent antibodies against the viral component of the modified spike glycoprotein (MoMLV envelope glycoprotein, for example) encoded by the viral nucleic acid and positively staining cells could be isolated by fluorescence-activated cell sorting (FACS). FACS would also facilitate the selection of cells on the basis of the level of expression of the transferred nucleic acid. Fluorescent staining of cells for expression of the MoMLV envelope spike glycoprotein has previously been demonstrated.

### 6. The mechanism of recovery from the infected target cells of the delivered nucleic acid which encodes the displayed polypeptide

The recovery of the nucleic acid sequences encoding the virus-displayed nonviral peptide, polypeptide or glycopolypeptide after the delivery of the nucleic acid to the a target cell can be achieved by PCR amplification using flanking oligonucleotide primers. PCR amplification may be from whole cells, from high molecular weight DNA or low molecular weight DNA extracted from the cells or from cDNA prepared from RNA extracted from the cells. Alternatively, the viral nucleic acid could be amplified and recovered directly from the infected target cells into progeny viruses by superinfection with wild-type virus, by transfection of a suitable helper plasmid (e.g. encoding the retroviral gag and pol proteins), or by using a library of recombinant viruses encapsidating a full-length infectious viral genome inclusive of the sequences encoding the displayed polypeptide.

The invention will now be further described by way of illustrative example and with reference to the accompanying drawings, in which:

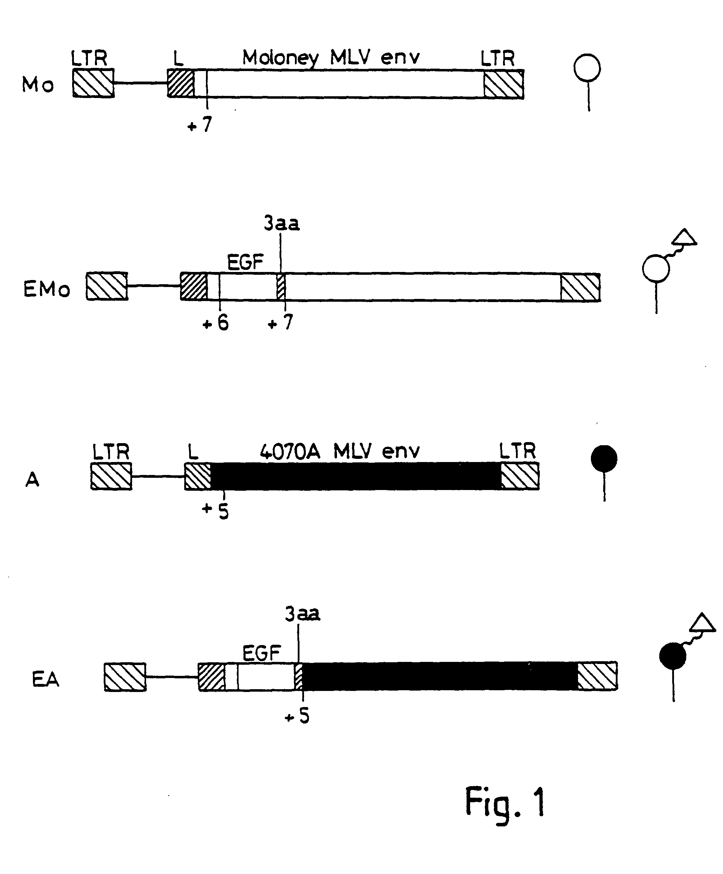

Figure 1 is a schematic representation of various wild type and chimaeric retrovirus envelope constructs; (EGF = epidermal growth factor, L = Leader signal sequence from Moloney MLV env; all env genes were expressed using the same promoter, the Friend MLV long terminal repeat "LTR", codon numbering refers to distance from N-terminus of mature SU glycoprotein);

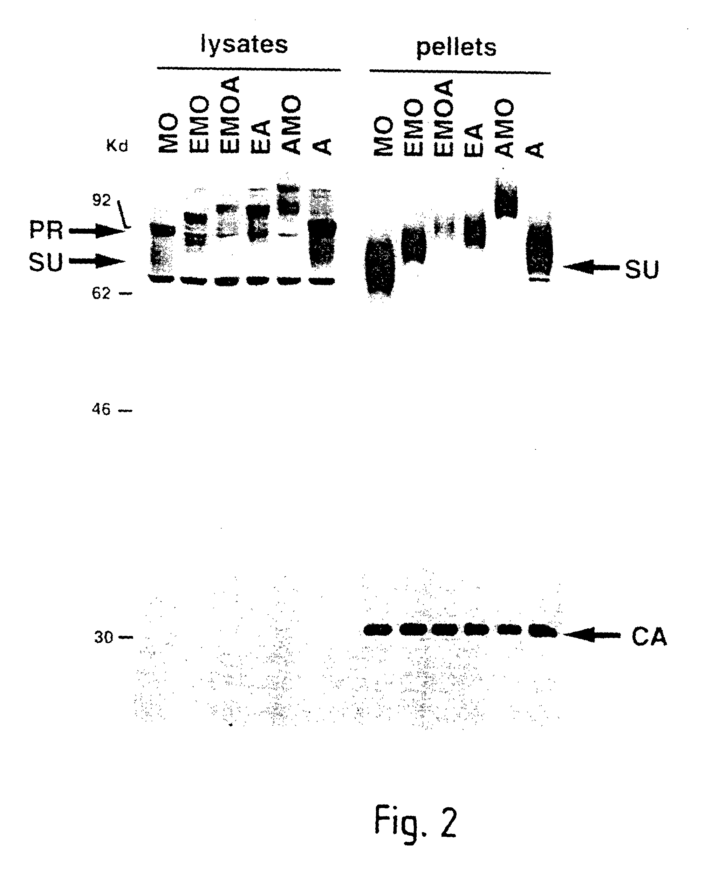

Figure 2 is a picture of Western blots, showing detection of envelope SU proteins; immunoblots of lysates (on the left) of TELCeB6 cells transfected with the envelopes shown in Figure 1 or of pellets (on the right) of viral particles produced from these cells. Both blots were stained with an SU anti-serum. The immunoblot of pellets was cut at 46 kD, and the lower part was stained with a p30-CA anti-serum;

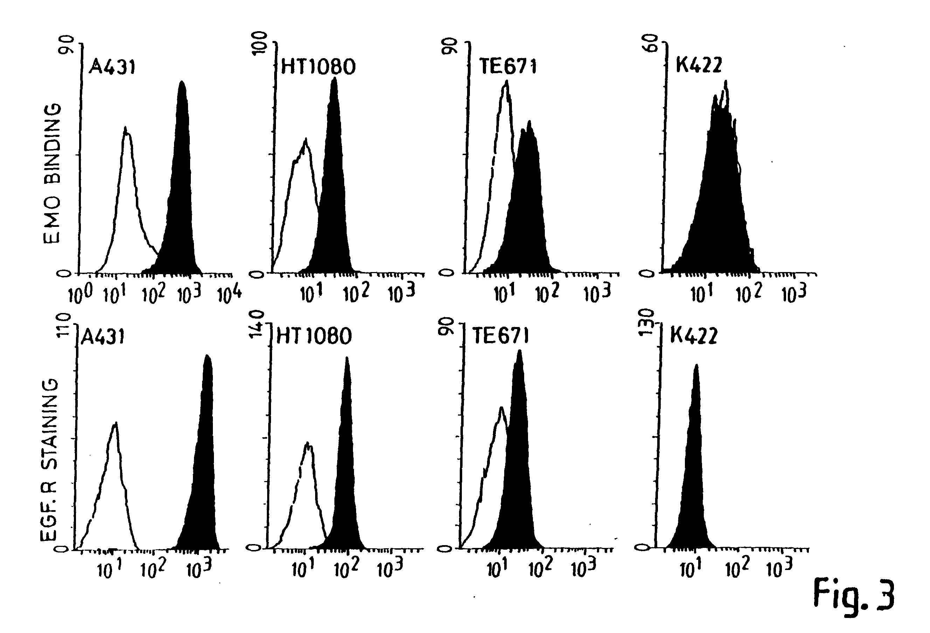

Figure 3 shows a series of graphs produced by fluorescence-activated cell sorting (FACS), illustrating the results of EGF receptor binding assays; cells were A431, HT1080, TE671, or K422. The top row shows binding assays performed using EMO envelopes (black histograms) compared to MO envelopes (white histograms), whilst the bottom row shows the results when the cells were stained with an anti-hEGF.R antibody;

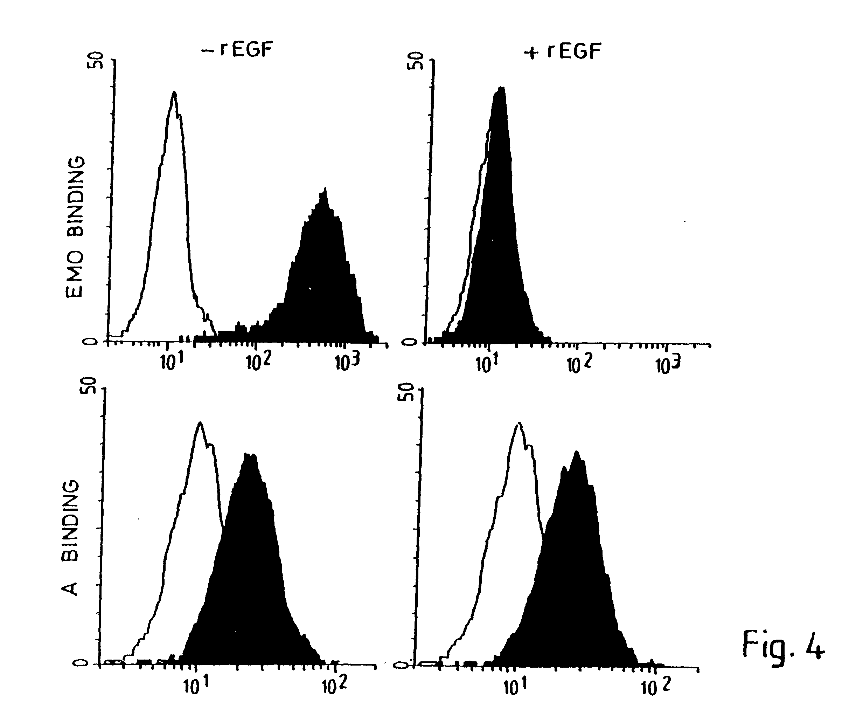

Figure 4 shows a series of graphs, produced by FACS, illustrating the specificty of EGF binding; A431 cells were used as target cells. Cells were (+ rEGF) or were not (-rEGF) treated with recombinant EGF (10⁻⁶ M, 30 min, 37°C) prior to binding assays using EMO (top row of graphs) or A envelopes (bottom row);

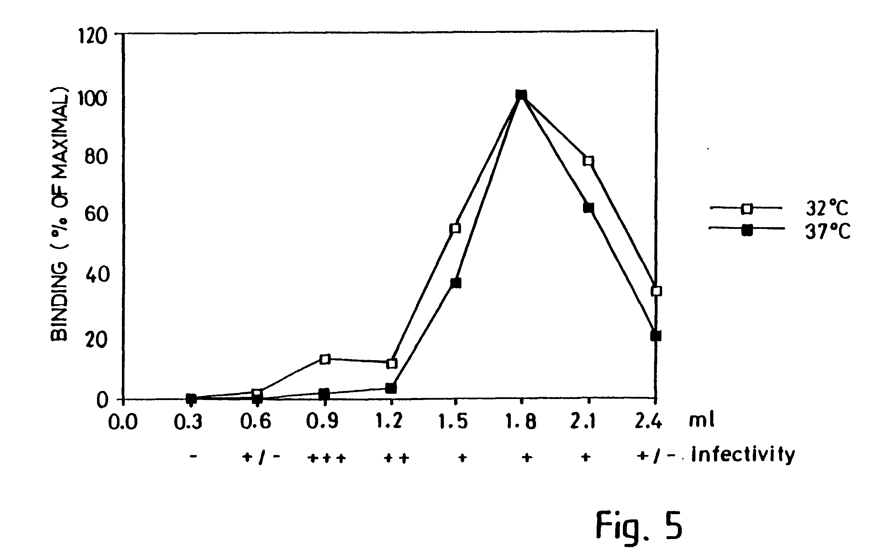

Figure 5 is a graph showing EGF binding of various fractions (see text) after S-1000 chromatography. Each fraction was analysed both for its binding activity using A431 cells as targets and for infectivity on 3T3 cells: (-): no infectivity, (+/-): 1-10 lacZ-EFU, (+): 10-100 lacZ-EFU, (++): 100-1000 lacZ-EFU, (+++): > 1000 lacZ-EFU;

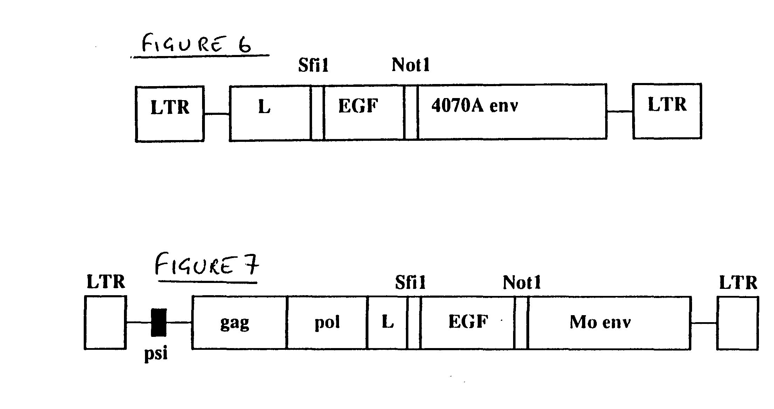

Figure 6 is a schematic representation of the chimeric envelope construct E.A, which comprises the EGF coding sequence inserted at codon +1 of the amphotropic 4070A envelope protein; and
Figure 7 is a schematic representation of the construct pNCAEGFL.1*Sfi*I replicating retroviral expression construct containing a *psi* packaging sequence.

### Examples

In an attempt to target retroviral gene delivery through the human epidermal growth factor receptor (EGFR) to EGFR-positive cells, the inventors and their colleagues constructed a chimaeric envelope construct in which the human EGF coding sequences were cloned, in frame, into a *Sfi*I - *Not*I cloning site which had been introduced between the 6th and 7th codons of the *env* gene of Moloney MLV (Russell *et al*, 1993 Nucleic Acids Research *21*, 1081-1085; Russell *et al*, WO94/06920). This chimaeric envelope construct (EMO) was then transfected into the complementing cell line TELCeB6 which expresses MLV gag and pol functions and a packagable RNA transcript coding for β-galactosidase. The transfected TELCeB6 cells released retroviral particles which efficiently incorporated the chimaeric EMO envelope glycoprotein, as shown by western blot analysis of pelleted virus. Viruses incorporating the chimaeric EMO envelope glycoprotein were shown by chromogenic substrate assay to transfer the β-galactosidase gene to mouse NIH3T3 cells, indicating that the chimaeric protein could still bind to the ecotropic MLV receptor and catalyse membrane fusion.

Viruses incorporating the chimaeric EMO envelope glycoprotein were then separated from soluble (shed) envelope glycoprotein by sephacryl column chromatography of 0.45µM-filtered culture supernatant and were shown by immunofluorescence staining to bind efficiently to EGF.R-positive human cell lines (i.e. cell lines expressing EGF receptor). Binding to these cells was competitively inhibited by soluble EGF. However, the bound virus did not transfer a functional β-galactosidase gene to the human cell lines, whereas control viruses incorporating the amphotropic 4070A envelope efficiently transferred the β-galactosidase gene to these same human cell lines.

The inventors and colleagues next examined the efficiency of gene transfer to EGFR-positive human cells using recombinant retroviruses displaying the EGF polypeptide fused to the amphotropic 4070A envelope glycoprotein. The chimaeric EGF-4070A (EA) expression construct was created by substituting most of the MoMLV env sequence in FBEGFMosA (Cosset *et al.*, J. Virol. 1995 **69**, 6314-6322), (from codon 7, immediately 3' of the *Not*I cloning site to the *Cla*I site close to the 3' end of the env gene) for the corresponding 4070A env sequence (from codon 5 to the *Cla*I site close to the 3' end of the env gene).

Recombinant retroviruses displaying the chimaeric EA envelope protein behaved in an unexpected fashion. They transferred the β-galactosidase gene efficiently to mouse NIH3T3 cells and to human cells that were negative for EGF receptor expression, but not to EGF receptor-expressing human cells. The titre reduction on EGF receptor-positive human cells was as much as ten million-fold compared to control viruses incorporating unmodified 4070A amphotropic envelopes.

Subsequent experiments using various target cells of mouse and human origin, some of which had been transfected with an EGFR expression plasmid, proved that viruses displaying the chimaeric EMO and EA envelope glycoproteins had selectively impaired ability to infect ecotropic or amphotropic MLV receptor-positive cells that also expressed EGF receptors. Higher levels of EGF receptor expression were associated with greater impairment of infectivity. Cells transfected with an EGF receptor expression plasmid became selectively resistant to EMO and EA viruses, and this resistance was reversible upon treating the cells with soluble EGF to block/downregulate the EGF receptors. Thus, retroviral host range was selectively restricted to EGF receptor-negative cells by displaying a high affinity ligand for EGF receptors on the viral surface.

The present inventors and their colleagues thus discovered a novel biological phenomenon which they have called ligand-dependent, receptor-mediated viral sequestration - a method by which to restrict the host range of a MLV or MLV-based retroviral vector in a ligand-dependent fashion. The first step is to identify a polypeptide ligand which binds specifically to a cell surface marker present on nontarget cells but absent from the target cell population. This polypeptide is then fused (by genetic engineering) to the retroviral envelope protein such that the envelope protein to which it has been grafted remains substantially intact and capable of binding to its natural receptor, and the fused non-viral polypeptide ligand is displayed on the viral surface. The virus displaying the fused non-viral polypeptide ligand is then capable of multivalent attachment to the natural virus receptor and to the the cognate receptor for the non-viral ligand; attachment to the natural virus receptor leads to infection of the target cell, whereas attachment to the cellular receptor for the displayed non-viral ligand may not lead to infection of the target cell. Where the target cell expresses both species of receptor and attachment through the displayed non-viral ligand does not lead to infection, the two binding reactions (envelope protein to natural receptor and nonviral ligand to its cognate receptor) proceed in competition and the infectivity of the virus for the target cells is reduced in proportion to the efficiency with which the second binding reaction competes virus away from the natural virus receptor.

The degree to which gene transfer is inhibited will therefore be influenced by the relative affinities of the two binding reactions, the relative densities of the two receptors on the target cell surface, and the relative densities of the non-viral ligand and the intact envelope protein on the viral surface. Inhibition of gene transfer is additionally influenced by intrinsic properties of the receptor for the non-viral ligand, such as the distance it projects from the target cell membrane, its mobility within the target cell membrane and its half life on the cell surface after engagement of ligand. This method of host range restriction may be applicable to the membrane spike glycoproteins of other enveloped viruses, and to the attachment proteins of non-enveloped viruses such as the adenovirus fibre protein. Where an enveloped virus has multiple distinct membrane spike glycoproteins with differing binding specificities and fusogenic capabilities (eg paramyxiviridae, herpesviridae), the restriction of virus host range by this method may or may not require the modification of more than one of the glycoproteins.

### Example 1

**Construction of chimaeric retroviral envelopes.** The sequence coding for EGF (epidermal growth factor) was inserted in MLV (murine leukemia virus) env gene in a position corresponding to amino-acid +6 in the SU glycoprotein of MoMLV (Fig. 1). This position of insertion was previously shown to allow the functional display of single chain antibodies at the surface of virions (Russell *et al*, 1993 NAR 21 p1081-1085). The EGF domain was separated from the wild type receptor binding domain in the envelope by a small linker containing 3 alanine residues. In the chimera EMO, EGF was inserted in the Mo-MLV envelope, whereas chimera EA had an EGF insertion in the MLV amphotropic (4070A) envelope at position +5. Envelopes, including the control envelopes from ecotropic (MO) and amphotropic (A) MLV, were transfected into TELCeB6 cells which express MLV gag-pol core particles and a lacZ retroviral vector.

**Expression and viral incorporation of chimaeric envelopes.** Lysates of TELCeB6 cells were analysed for envelope expression using antibodies against MLV SU (Fig. 2). For both chimeric envelopes, both a precursor and a processed SU product were detected at ratios similar to wild-type envelopes, suggesting that the mutants were correctly expressed and processed. Cell surface expression of mutant envelopes was examined by FACS analysis of producer cells, using antibodies against the SU or a monoclonal anti-hEGF antibody. All transfected cells were stained with the anti-SU antibodies, and cells expressing the EGF-fusion envelopes were stained with anti-EGF monoclonals (data not shown).

To demonstrate incorporation of the chimeric envelope glycoproteins into retroviral particles, supernatants of the various TELCeB6-transfected cell lines were ultracentrifuged to pellet viral particles. Pellets were then analysed on immunoblots for their content of gag (p30-CA) and envelope proteins (Fig. 2). The chimaeric SU glycoproteins were detected at a similar ratio to gag compared to wild-type envelope.

**Binding of chimaeric envelopes to EGF receptors.** Human cell lines expressing different numbers of EGF receptors (Fig. 3 bottom) were used for binding assays. Cells were incubated with virus supernatants and binding of viral envelopes to the target cell surface was analysed by FACS using antibodies against the MLV SU.

MoMLV-derived EGF-fusion envelopes (EMO envelopes) were found to bind to A431 cells (Fig. 3 top) over-expressing EGF.R (Fig. 3 bottom). Less binding was found on TE671 and HT1080 target cells which express less EGF.R (Fig. 3). No binding could be detected on K422 lymphoma cells with no detectable expression of EGF receptor (Fig. 3). The EA envelopes bound to A431 cells as well as EMO (data not shown). EGF receptors on A431 cells were down-regulated by pre-incubation with EGF. This treatment did not affect the binding of amphotropic envelopes (Fig. 4 bottom) but abolished binding of EMO envelopes (Fig. 4 top).

SU envelope glycoproteins of MLVs are known to be weakly associated with their TM protein counterparts (Gliniak *et al*, 1991 J Biol Chem 266 p22991-22997) and a very low proportion of SU is retained on virions. Therefore it is likely that binding assays in Fig. 3 are due in part to soluble envelope glycoproteins shed from virions. To determine whether viral particles could also bind, the supernatant of producer cells was separated by gel-filtration and fractions were analysed for binding activity on A431 cells (Fig. 5). As expected, very little binding activity was found in the early fractions containing the viral particles, with most of the binding activity in the late fractions containing soluble envelopes. However when viral particles were produced at 32°C (in order to reduce the dissociation between SU and TM) a significant binding activity was also found in the fractions containing the virions (Fig. 5), demonstrating that viral particles could bind EGF.R.

**Host range properties of viruses carrying EMO envelopes.** Table 1A shows that viruses incorporating EMO envelopes can infect NIH3T3 mouse fibroblasts. Infection is through the ecotropic MLV receptor because the EMO virus cannot infect NIH3T3 cells expressing the Moloney envelope glycoprotein but can infect those expressing the 4070A envelope glycoprotein. Viruses incorporating EMO envelopes can not only bind to EGF receptors but can also bind and infect cells through ecotropic MLV receptors.

Table 1B shows that viruses incorporating EMO envelopes could not infect human cells expressing various densities of EGF receptors, despite their ability to bind to the EGF receptors on these cells (Fig 3A). Surprisingly, the cell line EJ.A1, which stably expresses ecotropic MLV receptors from a transfected plasmid, could not be infected by the EMO virus, but was readily infected by viruses incorporating unmodified MO envelopes. This result suggested that the EMO virus could be competitively sequestered by EGF receptors on EJ.A1 cells, preventing it from binding to the ecotropic viral receptors.

**Table 1.**

| ***Infection by virions expressing targeting envelopes*** | | | |
|---|---|---|---|
| ***A. On mouse fibroblasts***^{***b***} | | | |
| ***env***^{***a***} | ***3T3*** | ***3T3*/*E*** | ***3T3*/*A*** |
| ***A 10***^{***7***} | ***10***^{***7***} | ***10***^{***2***} | |
| ***MO*** | ***10***^{***7***} | ***<1*** | ***10***^{***7***} |
| ***EMO*** | ***10***^{***5***} | ***<1*** | ***10***^{***5***} |
| ***EA 10***^{***6***} | ***nd*** | ***10***^{***1***} | |

**Table 1B.**

| ***On human cell lines***^{***b***} | | | | | | | |
|---|---|---|---|---|---|---|---|
| ***env***^{***a***} | ***A431*** | ***HT108*** | ***TE671*** | ***K422*** | ***Jurkat*** | ***EJ*** | ***EJ.A1*** |
| ***EGFR*** | ***++++*** | ***++*** | ***+*** | ***-*** | ***-*** | ***++*** | ***++*** |
| ***A*** | ***10***^{***7***} | ***10***^{***7***} | ***10***^{***7***} | ***10***^{***5***} | ***10***^{***4***} | ***10***^{***6***} | ***10***^{***6***} |
| ***MO*** | ***<1*** | ***<1*** | ***<1*** | ***<1*** | ***<1*** | **<*1*** | ***10***^{***6***} |
| ***EMO*** | ***<1*** | ***<1*** | ***<1*** | ***<1*** | ***<1*** | ***<1*** | ***<1*** |
| ***EA*** | ***<1*** | ***<1*** | ***10***^{***1***} | ***10***^{***4***} | ***10***^{***3***} | ***<1*** | ***<1*** |

| | | | | | | | |
|---|---|---|---|---|---|---|---|
| ***a: envelope expressed on lacZ virions*** | | | | | | | |
| ***b: titres as lacZ-EFU*/*ml. Abbreviations for cell lines: 3T3: NIH3T3; 3T3*/*E: psi2; 3T3*/*A: GP+EAM12*** | | | | | | | |

**Table 2.**

| ***Inhibition of infection by EGF.R*** | | | |
|---|---|---|---|
| ***A. NR6 cells***^{***b***} | | | |
| | ***NR6 .*** | ***NR6-wt hEGF.R .*** | |
| | | ***- rEGF***^{***c***} | ***+ rEGF***^{***c***} |
| ***enV***^{***a***} | ***titre*** | ***titre*** | ***titre*** |
| ***MO*** | ***1x10***^{***6***} | ***5x10***^{***5***} | ***5x10***^{***5***} |
| ***EMO*** | ***5x10***^{***4***} | ***1x10***^{***3***} | ***10***^{***5***} |
| ***A*** | ***7x10***^{***4***} | ***2x10***^{***5***} | ***2x10***^{***5***} |
| ***EA*** | ***2x10***^{***5***} | ***3x10***^{***3***} | ***5x10***^{***5***} |

| ***B. NIH3T3 cells***^{***b***} | | | |
|---|---|---|---|
| ***EGFR No*.** | ***10,000*** | ***80*,*000*** | ***400,000*** |
| ***env***^{***a***} | ***titre*** | ***titre*** | ***titre*** |
| ***MO*** | ***10***^{***6***} | ***10***^{***6***} | ***10***^{***6***} |
| ***EMO*** | ***10***^{***5***} | ***10***^{***4***} | ***10***^{***3***} |

| | | | |
|---|---|---|---|
| ***a: envelope expressed on lacZ virions*** | | | |
| ***b: titers* as *lacZ-EFU*/*ml.*** | | | |
| ***c: cells were (+) or were not (-) pre-incubated with 10***^{**-4**} ***M recombinant EGF for 30 min at* 37°C** | | | |

**Competitive sequestration of viruses carrying EMO envelopes.** To test the idea that EMO viruses could be competitively sequestered by EGF receptors at the surface of an otherwise permissive target cell, we titrated viruses incorporating EMO or MO envelopes on mouse fibroblasts (NR6 and NIH3T3) expressing variable numbers of EGF receptors (Table 2). The titers of viruses carrying EMO envelopes were reduced up to 1000-fold by EGF.R expression and there was a correlation between the density of EGF receptor expression and the magnitude of reduction in virus titre (Table 2B). When NR6-hEGF.R cells were pre-treated with rEGF, which down-regulates EGF.R as confirmed by antibody staining (not shown), titers of viruses coated with EGF-fusion envelopes were greatly enhanced, reaching the range of titers obtained on parental NR6 cells (Table 2A). These data confirm that interaction of virions with EGF receptors leads to their sequestration into an abortive entry pathway that does not lead to membrane fusion or cytoplasmic release of the viral cores.

**Host range and competitive sequestration of viruses carrying EA envelopes.** Table 1A shows that viruses incorporating EA envelopes can infect NIH3T3 mouse fibroblasts through the amphotropic MLV receptor. When titrated on a panel of human cell lines expressing varying densities of EGF receptors, the EA virus showed a selective inability to infect all of the EGF receptor-positive human cells in the panel (Table 1B). However, they could easily infect human B and T cell lines (K422 and Jurkat) which are devoid of EGF.R and are presumably infected through the amphotropic receptor (Table 1). These data suggested that the EA viruses were efficiently sequestered by EGF.R expressed on human cells and to confirm their competitive sequestration, they were tested on parental and EGF receptor-expressing NR6 mouse fibroblasts (Table 2A). EGF receptor expression on NR6 cells led to a competitive inhibition (100-fold) of viral infection which was reversible when the NR6 transfectants were pre-treated with EGF to block/downregulate their EGF receptors.

**Some sequestered virus can be rescued into an infectious entry pathway.** After binding to receptor, EGF induces receptor dimerisation and its signal transduction, followed by ligand-receptor internalisation and routing to lysosomes, where EGF-EGF receptor complexes are degraded (Carpenter & Cohen, 1990 J. Biol. Chem. 265 p7709-7712). We suspected that viruses bound to EGF receptors might therefore be rapidly internalised into the cell and routed to lysosomes for degradation. When EMO-carrying viral particles were used to infect A431 cells treated with the inhibitor of lysosomal degradation, chloroquine, a significant increase of infectivity (by approximately 2 logs) was obtained (Table 3). This effect was specific to EGF.receptors as EGF.receptor negative cells, such as K422 cells, did not respond similarly (Table 3).

**Table 3.**

| ***Effect of chloroquine on infection*** | | | | | | | | |
|---|---|---|---|---|---|---|---|---|
| | ***NIH3T3***^{***b***} ***.*** | | ***A431***^{***b***} ***.*** | | ***TE671***^{***b***} ***.*** | | ***K422***^{***b***} ***.*** | |
| ***enV***^{***a***} | ***-*** | ***+*** | ***-*** | ***+*** | ***-*** | ***+*** | **-** | ***+*** |
| ***MO*** | ***10***^{***6***} | ***5x10***^{***5***} | ***<1*** | ***6*** | ***<1*** | ***1*** | ***<1*** | ***<1*** |
| ***EMO*** | ***10***^{***5***} | ***5x10***^{**4**} | ***1*** | ***225*** | ***<1*** | ***46*** | ***<1*** | ***<1*** |

| | | | | | | | | |
|---|---|---|---|---|---|---|---|---|
| ***a: envelope expressed on lacZ virions*** | | | | | | | | |
| ***b: titres* as lacZ *EFU*/*ml.* Cells *were treated (+) or not (-) with chloroquine.*** | | | | | | | | |

### Materials and Methods

**Cell lines.** TELCeB6 cell line was derived from the TELac2 line (Takeuchi *et al*, 1994 J. Virol. 68 p8001-8007) after transfection and clonal selection of cells containing a plasmid expressing MoMLV (Moloney murine leukemia virus) gag and pol proteins. TELCeB6 cells produce non-infectious viral core particles, carrying the MGFnlsLacZ reporter retroviral vector (Ferry *et al*, 1991 Proc. Natl. Acad. Sci. USA 88 p8377-8381). A431, TE671 (ATCC CRL8805), HT1080 (ATCC CCL121), EJ (Bubenik *et al*, 1973 Int. J. Cancer 11 p765-773) and EJ.A1, (an EJ clone that expresses ecotropic MLV receptors, Albritton *et al*, 1989 Cell 57 p659-666) were grown in DMEM (Gibco-BRL) supplemented with 10% fetal bovine serum (Gibco-BRL). K422 cells (Dyer *et al*, 1990 Blood 75 p709-714) and Jurkat T cells were grown in RPMI 1640 (Gibco-BRL) supplemented with 10% fetal bovine serum (Gibco-BRL). NR6 murine fibroblasts lacking detectable EGF receptors (Schneider *et al*, 1986 Proc. Natl. Acad. Sci. USA 83 p333-336) and NR6-EGF.R (an NR6 subclone obtained after transfection of a plasmid expressing the human epidermal growth factor receptor) cells were kindly provided by G. Gill (La Jolla, USA). psi2 cells (Mann *et al*, 1983 Cell 33 p153-159) and GP+EAM12 cells (Markowitz *et al*, 1988 Virol. 167 p400-406) were derived from NIH-3T3 cells and express respectively MoMLV (ecotropic) and MLV-amphotropic envelopes which block the corresponding receptors (EcoR-1 and RAM-1) by interference. NIH3T3 clones transfected with EGF receptor expression constructs and expressing moderate or high levels of EGF receptors were kindly provided by Prof Thierry Velu (Erasmus Hospital, Brussels). NIH-3T3 and NIH-3T3-derived cell lines were grown in DMEM (Gibco-BRL) supplemented with 10% new born bovine serum (Gibco-BRL).

**Chimeric envelopes.** A PCR-derived DNA fragment encoding the 53 aa of hEGF (Bell *et al*, 1986 Nucleic Acids Res. 14 p8427-8446) was generated using a cDNA template (ATCC 59957) and two primers: (Seq. ID No. 1) with an *Sfi*I restriction site, and (Seq. ID No. 2) with a *Not*I site, and cloned after digestion with *Sfi*I and *Not*I in either MoMLV SU for the EMO chimeric envelope or 4070A SU for the EA envelope (Figure 1).

All envelope constructs were expressed as *Bgl*II*-Cla*I fragments (corresponding to positions 5408 and 7676 in MoMLV), cloned between *Bam*HI and *Cla*I sites of the FBMOSALF expression vector (Cosset *et al*, *J.* Virol. 1995 **69**, 6314-6322), in which a phleo selectable marker (Gatignol *et al*, 1988 FEBS Lett. 230 p171-175) fused to the PGK (phospho-glycerate kinase) gene poly-adenylation sequence was introduced downstream to the C57 MLV LTR of FB3 (Battini *et al*, 1992 J. Virol. 66 p1468-1475).

**Production of viruses.** Envelope expression plasmids were transfected by calcium phosphate precipitation into TELCeB6 cells. Transfected cells were selected with phleomycin (50 mg/ml) and pools of phleomycin-resistant clones were used to harvest viruses from confluent cells after overnight incubation in DMEM and FBS (10%). These supernatants were used for ultracentrifugation to provide Western blot samples, for binding assays and for infection assays. Viruses (in 100 ml of producer cell supernatant) were also purified by gel-filtration on 2 ml columns (Bio-Rad) on a bed of S-1000 Sephacryl (Pharmacia). Fractions were obtained by elution with PBS at 4°C.

**Immunoblots.** Virus producer cells were lysed in a 20 mM Tris-HCl buffer (pH 7.5) containing 1% Triton-X100, 0.05% SDS, 5 mg/ml sodium deoxycholate, 150 mM NaCl, and 1 mM PMSF. Lysates were incubated for 10 min at 4°C and were centrifuged for 10 min at 10,000xg to pellet the nuclei. Supernatants were then frozen at -70°C until further analysis. Virus samples were obtained by ultracentrifugation of viral supernatants (10 ml) in a SW41 Beckman Rotor (30,000 RPM, 1 hr, 4°C). Pellets were suspended in 100 µl of PBS (phosphate buffered saline), and frozen at -70°C. Samples (30 mg for cell lysates, or 10 µl for purified viruses) were mixed in a 375 mM Tris-HCl (pH 6.8) buffer containing 6% SDS, 30% b-mercapto-ethanol, 10% glycerol, and 0.06% bromophenol blue, boiled for 3 min, then run on 10% SDS acrylamide gels. After protein transfer onto nitrocellulose filters, immunostaining was performed in TBS (Tris base saline, pH 7.4) with 5% milk powder and 0.1% TWEEN. Antibodies (Quality Biotech Inc, USA) were goat antisera raised against either RLV (Rausher leukemia virus) gp70-SU protein or RLV p30-CA protein, and were diluted 1/1,000 and 1/10,000, respectively. Blots were developed using HRPO-conjugated rabbit anti-goat antibodies (DAKO, UK) and an electrochemiluminescence kit (Amersham Life Science).

**Binding assays.** Target cells were washed in PBS and detached by a 10 min incubation at 37°C with versene 0.02% in PBS. Cells were washed in PBA (PBS with 2% FCS and 0.1% sodium azide). 10⁶ cells were incubated with viruses for 30 min at 4°C. Cells were then washed with PBA and incubated in PBA containing 1/200 of RLV gp70 immune serum for 30 min at 4°C. Cells were washed twice with PBA and incubated with rabbit anti-goat FITC-conjugated antibodies (DAKO, U.K.). 5 min before the two final washes in PBA, cells were stained with 20 mg/ml propidium iodide. Fluorescence of living cells was analysed with a fluorescent-activated cell sorter (FACSCan, Beckton Dickinson). For hEGF.R staining, 10⁶ cells in 100 ml of PBA were incubated with 10 ml of anti-EGF.R antibodies (M886, DAKO, U.K.) for 30 min at 4°C.

**Infection assays.** Target cells were seeded in 24 multi-well plates at a density of 3x10⁴ cells per well or in 6-multi-well plates at a density of 2x10⁵ cells per well. Viral supernatant dilutions containing 4 mg/ml polybrene were added and cells were incubated for 3-5 hrs at 37°C. Viral supernatant was then removed and cells were incubated in regular medium for 24-48 hrs. X-Gal staining was performed as previously described (Takeuchi *et al*, 1994 J. Virol. 68 p8001-8007).

To block EGF.R, target cells were incubated 30 min at 37°C in a medium containing 10⁻⁶ M rEGF (236-EG, R&D Systems, U.K.). Cells were then washed and infections were carried out as previously described. To block lysosomal acidification, 100 mM chloroquine phosphate (Sigma, U.K.) was added to the medium for 6 hr from the start of the infection protocol after which the cells were washed and incubated in regular medium.

### Example 2: Making the plasmid library constructs

### a) PCR Mutagenesis and cloning into retroviral expression constructs

A library of EGF cDNA mutants was generated by random PCR mutagenesis. The chimaeric EGF amphotropic (E.A.) vector (Cosset *et al.* 1995 J. Virol 69 p6314), with an EGF cDNA inserted at +1 codon of the N terminus of the 4070A murine leukemia virus (MLV) surface protein (SU) gp 70 (Figure 6) and flanked by *Sfi*I and *Not*I restriction sites, was used as a template for PCR random mutagenesis (Hawkins *et al.* 1992 J. Mol. Biol. 226 p889). The 100 µl reaction mixture contained 20 ng template, 50 pmoles of Bglenvrev primer (5' TCT AGA CTG ACA TGG CGC GT, Seq. ID No. 3) and 4070Aseq3 primer (5' GGT GAC TCT CCA GGT TAC, Seq. ID No. 4), 1mM of each deoxynucleotide triphosphate, 50 mM KCl, 10 mM Tris-HCl (pH 9.0 at 25°C), 6 mM MgCl₂, 0.5 mM MnCl₂ and 1% (v/v) Triton X-100.

The MnCl₂ was added fresh just before the start of the PCR. Amplification consisted of 30 cycles of 94°C (1 min), 65°C (1 min) and 70°C (4 min) using Taq DNA polymerase (Promega; 5 units). The resulting PCR-generated cDNA fragments were digested with *Sfi*I and *Not*I restriction enzymes and cloned into *Sfi*I*-Not*I digested pNCAEGFL.1*Sfi*I-backbone (Figure 7) in which the *Sfi*I site in the pol gene was deleted by PCR mutagenesis without change to the amino acid sequence.

The plasmid library of pNCAEGFL. 1*Sfi*- retroviral expression constructs were transformed into electrocompetent *E. coli*. Thirty-five individual clones were picked, their respective DNA extracted and digested with *Sfi*I and *Not*I restriction enzymes. Only clones that gave a correct fragment size of about 159 base pairs (EGF cDNA size=159 bp) were selected and sequenced by automated sequencing.

### b) Sequencing

Twenty six clones of the pNCAEGFL.1*Sfi*I-plasmid library was sequenced to check for the number of mutations in the EGF gene. PCR cDNA fragments were generated from the DNA of each of the respective clones using Bglenvrev and envseq5 (5' GTA AGG TCA GGC CAC CAG GT, Seq. ID No. 5) primers at final concentration of 25 µmoles each, 5 µl Taq polymerase buffer (Promega), 0.25 mM dNTP and amplified for 25 cycles of 94°C (30s), 60°C (30s) and 72°C (30s) using Taq polymerase (5 units). The PCR fragments were gel purified and used as template for cycle sequencing with 9.5 µl of terminator ready reaction mix (ABI PRISM™) and 3.2 pmole of envseq5 primer.

*Results:* Out of 26 clones sequenced, 18 had a mutated EGF sequence with one to five point mutations in the codon (Table 4), making a total of 39 point mutations.

**Table 4**

| Number of clones with 1-5 point mutations in the EGF cDNA. | |
|---|---|
| **Number of mutations** | **Number of occurrences** |
| 0 | 9 |
| 1 | 7 |
| 2 | 4 |
| 3 | 5 |
| 4 | 1 |
| 5 | 1 |

Percentage of clones with 1-5 point mutations = 18 x 100/26 = 69%
Total number of bases sequenced = 4293
Total number of point mutations = 39
Number of mutations per gene (159 bases) = 39 x 159/4293 = 1.4

### c) Size of the pNCAEGFL.1SfiI-plasmid library

The PCR-generated EGF cDNA was cloned into the pNCAEGFL.1*Sfi*I-backbone. Ten different ligations were performed. The ligated products were transformed into electrocompetent cells (Stratagene, Epicurian coli XL1-MRF'), yielding 10⁵ transformed cells/ml. The transformed cells were selected in 50 mg/ml ampicillin. Five batches of transformants were grown on large (225 cm x 225 cm) 2 x TY plates while the rest were grown respectively in 5 x 500 ml of 2 x TY liquid media overnight at 37°C. The next day, colonies of cells on the plates were scraped off and resuspended in 6 ml 2 x TY/ampicillin. Cells from both plates and liquid media were pooled, pelleted and resuspended in 5 ml of 2 x TY/15% glycerol/1% glucose/50 mg/ml ampicillin. An aliquot (50 µl) of the mixture was used to set up a midiprep for extraction of DNA while the rest was frozen in liquid nitrogen until further analysis.

### Example 3: Generation of the retroviral display library

### a) Making viral producer cells expressing Moloney MLV ecotropic receptor (Rec-1)

The human fibrosarcoma cell line, HT1080, was transfected with an expression vector coding for the Moloney MLV ecotropic receptor, Rec-1. Expression vectors were constructed by blunt end ligation of the cDNA fragment coding for Rec-1 into the RC/CMV vector at the *Not*I site of the polylinker, detailed below. The RC/CMV Rec-1 expression vector was stably transfected into HT1080 cells by the calcium phosphate precipitation method and the cells were pooled and expanded in 10% FCS-DMEM selection media containing 1 mg/ml neomycin (G418). The expression of the Rec-1 receptor was confirmed by checking if the HT1080-Rec-1 cells could be infected by wild type MoMLV virus transferring the β-galactosidase gene. About 65% of the transfected cells were stained blue with X-gal.

The polylinker in RC/CMV Rec-1 expression vector contains the following sites:
*Pcm*v/*Kpn*I/*Sac*I/*BamH*I/*Spe*I/*Xma*III/*BstX*I/*EcoR*I/*Pst*I/*ECOR*V/*BstX*I/*Not*I/*Xho*I/*Sph*I/ *Nsi*I/*Xba*I/*Hind*III/*Cla*I/....

### b) Generation and selection of retroviral library

The EGF plasmid library is transiently transfected into HT1080 cells by calcium phosphate precipitation method (Sambrook *et al.*, Molecular Cloning, Cold Spring Harbour Laboratory Press). After 72 h the viral supernatant from the HT1080 cells, which contains EGF displaying replicating vectors, is harvested overnight in 5 ml 10% FCS-DMEM and filtered (0.45 µm). It is then used to infect HT1080-Rec-1 cells in the presence of 4 µg/ml polybrene. After 72 h, the media from infected HT1080-Rec-1 cells are removed and replaced with 5ml 10%FCS-DMEM. The viral supernatant is harvested as before and used to infect a fresh batch of HT1080-Rec-1. A total three rounds of selection are carried out.

### Example 4: Characterisation of the Selected retroviral library

### a) Extraction of high molecular weight DNA from HT1080-Rec-1 cells

High molecular weight DNA is extracted from the infected HT1080-Rec-1 cells from both the second and third rounds of selection. The cells are washed with PBS, incubated in DNA lysis buffer (TE buffer, 1% SDS) containing 100 µg/ml proteinase K at 37°C for 5 minutes. Cell lysates are incubated at 37°C overnight and then extracted once in 2 ml phenol, by mixing it gently on a rotor wheel for 15 minutes, centrifuged for 15 minutes at 1500xg and then the aqueous phase is transferred to a new tube. The process is repeated once with phenol/chloroform (1:1) and then with chloroform only. After the final chloroform extraction, 2.5 volumes salt/ethanol (250 vol. ethanol: 1 vol. 3M KAc at pH 4.8) is added and the solution gently rocked till the DNA formed a contracted, cottonwool-like precipitate. The DNA is then spooled out using a blunt glass hook, drained for a few seconds and allowed to dissolve overnight in 200 µl TE.

### b) PCR amplification of EGF cDNA from high molecular weight DNA

Polymerase chain reaction is carried out as before using Bglenvrev and envseq5 primers on a diluted sample of the high molecular weight DNA to amplify the EGF cDNA. The PCR product is ligated into TA Cloning vector (Invitrogen) and the vector is transformed into competent cells. Positive colonies containing the insert are picked and DNA from the individual colonies are extracted and the sequences of the EGF insert are checked on an automated sequencer. Mutations identified in this way are presumed to reduce the affinity of binding to EGF receptors but not to interfere with the folding of the EGF domain. This is confirmed by amplifying the mutant EGF cDNA fragment by PCR and cloning it into the EGF chimaeric construct (E.A). The construct is then transfected into the viral producer cells (TELCeB6) and the viral supernatant harvested for infection assays.

## Claims

1. A method of screening a plurality of altered first members of a specific binding pair ("sbp") for reduced ability to bind to the second member of the sbp compared to an unaltered first member; the method comprising introducing a plurality of nucleic acid sequences, each encoding a respective altered first member of the sbp, into a plurality of viral display packages so as to form a viral display package library in which each viral display package displays on its surface an altered first member of the sbp encoded by a respective nucleic acid sequence comprised within the viral display package and an infection-competent binding moiety capable of mediating infection of a susceptible target cell; contacting the viral display packages with a plurality of target cells which express on their surface a receptor molecule for the infection-competent binding moiety, such that binding to the receptor molecule tends to facilitate infection of a target cell by the viral display package, said target cells also expressing on their surface the second member of the sbp, binding to which, via the displayed altered first member of the sbp, tends to inhibit infection of a target cell by the viral display package, such that those viral display packages displaying altered first members with reduced ability to bind to the second member of the sbp will tend to infect target cells more successfully; and recovering from infected target cells or their progeny nucleic acid comprising those nucleic acid sequences which encode altered first members with reduced ability to bind to the second member of the sbp.

2. A method according to claim 1, wherein the viral display package is capable of infecting eukaryotic cells.

3. A method according to claim 1 or 2, wherein the viral display package is based on a retrovirus.

4. A method according to any one of claims 1, 2 or 3, wherein the viral display package is based on a C type retrovirus.

5. A method according to any one of the preceding claims, wherein the first member of the sbp is displayed as a fusion polypeptide.

6. A method according to any one of the preceding claims, wherein the first member of the sbp is displayed as a fusion with the infection-competent binding moiety.

7. A method according to any one of the preceding claims, wherein the first member of the sbp is displayed as a fusion with a viral envelope protein.

8. A method according to any one of the preceding claims, wherein the first member of the sbp is displayed as an N-terminal fusion with a retroviral envelope glycoprotein.

9. A method according to any one of the preceding claims, wherein the infection-competent binding moiety comprises a substantially intact retroviral envelope glycoprotein.

10. A method according to any one of the preceding claims, wherein the target cell is eukaryotic.

11. A method according to any one of the preceding claims, wherein the target cell is mammalian.

12. A method according to any one of the preceding claims, wherein the target cell naturally expresses the receptor molecule and the second member of the sbp.

13. A method according to any one of the preceding claims, wherein the target cell has been transfected with a sequence directing the expression of the receptor molecule and/or the second member of the sbp.

14. A method according to any one of the preceding claims, wherein the plurality of nucleic acid sequences encoding respective altered first members of the sbp are derived by the introduction of mutations into the sequence encoding the unaltered first member of the sbp.

15. A method according to claim 14, wherein the mutations are introduced by a process of substantially random mutagenesis.

16. A kit for use in performing the method of any one of the preceding claims, comprising a nucleic acid construct suitable for accepting a sequence encoding a first member of an sbp; retroviral packaging cells for producing viral display packages incorporating the nucleic acid construct; target cells expressing on their surface a receptor which allows infection by the viral display packages and also expressing the second member of the sbp; and instructions for performing a method in accordance with any one of the preceding claims.

17. A kit according to claim 16, further comprising one or more of the following: means for introducing mutations in the nucleic acid sequence encoding the unaltered first member of the sbp; and detection means for detecting infection of a target cell by a viral display package.

## Patentansprüche

1. Verfahren des Durchsuchens einer Vielzahl veränderter erster Mitglieder eines spezifischen Bindungspaars ("sbp") auf im Vergleich zu einem unveränderten ersten Mitglied verringerte Fähigkeit, an das zweite Mitglied des sbp zu binden, wobei das Verfahren das Einführen einer Vielzahl jeweils ein verändertes erstes Mitglied des sbp codierender Nukleinsäuresequenzen in eine Vielzahl von viralen Display-Paketen, womit eine virale Display-Paketbibliothek gebildet wird, in der jedes virale Display-Paket ein von einer in dem viralen Display-Paket enthaltenen jeweiligen Nukleinsäure codiertes verändertes erstes Mitglied des sbp und eine infektionskompetente Bindungseinheit, die die Infektion einer infizierbaren Zielzelle vermitteln kann, auf seiner Oberfläche präsentiert; das Inkontaktbringen der viralen Display-Pakete mit einer Vielzahl von Zielzellen, die auf ihrer Oberfläche ein Rezeptormolekül für die infektionskompetente Bindungseinheit exprimieren, so daß durch Bindung an das Rezeptormolekül die Infektion einer Zielzelle mit dem viralen Display-Paket erleichtert werden kann, wobei die Zielzellen auch das zweite Mitglied des sbp auf ihrer Oberfläche exprimieren und durch Bindung des präsentierten, veränderten ersten Mitglieds des sbp an dieses die Infektion einer Zielzelle mit dem viralen Display-Paket gehemmt werden kann, so daß solche viralen Display-Pakete, die veränderte erste Mitglieder mit verringerter Fähigkeit, an das zweite Mitglied des sbp zu binden, präsentieren, Zielzellen erfolgreicher infizieren werden können; und das Zurückgewinnen von Nukleinsäure, die solche Nukleinsäuresequenzen enthält, die veränderte erste Mitglieder mit verringerter Fähigkeit, an das zweite Mitglied des sbp zu binden, codieren, aus infizierten Zielzellen oder deren Nachkommen umfaßt.

2. Verfahren nach Anspruch 1, wobei das virale Display-Paket eukaryotische Zellen infizieren kann.

3. Verfahren nach Anspruch 1 oder 2, wobei das virale Display-Paket auf einem Retrovirus basiert.

4. Verfahren nach einem der Ansprüche 1, 2 und 3, wobei das virale Display-Paket auf einem Retrovirus vom Typ C basiert.

5. Verfahren nach einem der vorhergehenden Ansprüche, wobei das erste Mitglied des sbp als Fusionspolypeptid präsentiert wird.

6. Verfahren nach einem der vorhergehenden Ansprüche, wobei das erste Mitglied des sbp als Fusion mit der infektionskompetenten Bindungseinheit präsentiert wird.

7. Verfahren nach einem der vorhergehenden Ansprüche, wobei das erste Mitglied des sbp als Fusion mit einem viralen Hüllprotein präsentiert wird.

8. Verfahren nach einem der vorhergehenden Ansprüche, wobei das erste Mitglied des sbp als N-terminale Fusion mit einem retroviralen Hüllglycoprotein präsentiert wird.

9. Verfahren nach einem der vorhergehenden Ansprüche, wobei die infektionskompetente Bindungseinheit ein weitgehend intaktes retrovirales Hüllglycoprotein umfaßt.

10. Verfahren nach einem der vorhergehenden Ansprüche, wobei es sich bei der Zielzelle um eine eukaryotische Zelle handelt.

11. Verfahren nach einem der vorhergehenden Ansprüche, wobei es sich bei der Zielzelle um eine Säugerzelle handelt.

12. Verfahren nach einem der vorhergehenden Ansprüche, wobei die Zielzelle das Rezeptormolekül und das zweite Mitglied des sbp auf natürliche Weise exprimiert.

13. Verfahren nach einem der vorhergehenden Ansprüche, wobei die Zielzelle mit einer Sequenz, die die Expression des Rezeptormoleküls und/oder des zweiten Mitglieds des sbp steuert, transfiziert ist.

14. Verfahren nach einem der vorhergehenden Ansprüche, wobei die Vielzahl jeweilige veränderte erste Mitglieder des sbp codierender Nukleinsäuresequenzen durch die Einführung von Mutationen in die das unveränderte erste Mitglied des sbp codierende Sequenz abgeleitet wird.

15. Verfahren nach Anspruch 14, wobei die Mutationen durch einen weitgehend zufälligen Mutageneseprozess eingeführt werden.

16. Kit zur Verwendung bei der Durchführung des Verfahrens eines der vorhergehenden Ansprüche, umfassend ein zur Aufnahme einer ein erstes Mitglied eines sbp codierenden Sequenz geeignetes Nukleinsäurekonstrukt; retrovirale Verpackungszellen zur Herstellung das Nukleinsäurekonstrukt enthaltender viraler Display-Pakete; Zielzellen, die auf ihrer Oberfläche einen Rezeptor, der Infektion durch die viralen Display-Pakete gestattet, und auch das zweite Mitglied des sbp exprimieren; und Anweisungen zur Durchführung eines Verfahrens gemäß einem der vorhergehenden Ansprüche.

17. Kit nach Anspruch 16, weiterhin umfassend eines oder mehrere der Folgenden: Mittel zur Einführung von Mutationen in die das unveränderte erste Mitglied des sbp codierende Nukleinsäuresequenz; und Nachweismittel zum Nachweis der Infektion einer Zielzelle mit einem viralen Display-Paket.

## Revendications

1. Procédé de criblage d'une pluralité de premiers membres modifiés d'une paire spécifique de liaison ("psl") pour une aptitude réduite à lier le second membre de la psl en comparaison d'un premier membre non modifié ; le procédé comprenant l'introduction d'une pluralité de séquences d'acide nucléique, chacune codant pour un premier membre modifié respectif de la psl, dans une pluralité d'empaquetages à manifestation virale de façon à former une banque d'empaquetages à manifestation virale dans laquelle chaque empaquetage à manifestation virale présente sur sa surface un premier membre modifié de la psl codé par une séquence respective d'acide nucléique comprise dans l'empaquetage à manifestation virale et une partie de liaison capable d'infecter, apte à servir d'intermédiaire pour infecter une cellule cible sensible ; la mise en contact des empaquetages à manifestation virale avec une pluralité de cellules cible qui expriment sur leur surface une molécule réceptrice pour la partie de liaison capable d'infecter, de sorte que la liaison à la molécule réceptrice tend à faciliter l'infection de la cellule cible par l'empaquetage à manifestation virale, lesdites cellules cible exprimant également sur leur surface le second membre de la psl, la liaison auquel, via le premier membre de la psl modifié présenté, tend à inhiber l'infection d'une cellule cible par l'empaquetage à manifestation virale, de sorte que ces empaquetages à manifestation virale présentant des premiers membres modifiés à aptitude réduite à lier le second membre de la psl auront tendance à infecter les cellules cible avec plus de succès ; et la récupération, à partir des cellules cible infectées ou de leur progéniture d'acide nucléique comprenant ces séquences d'acides nucléiques qui codent pour des premiers membres modifiés à aptitude réduite à lier le second membre de la psl.

2. Procédé selon la revendication 1, dans lequel l'empaquetage à manifestation virale est capable d'infecter des cellules eucaryotiques.

3. Procédé selon la revendication 1 ou 2, dans lequel l'empaquetage à manifestation virale est basé sur un rétrovirus.

4. Procédé selon l'une quelconque des revendications 1, 2 ou 3, dans lequel l'empaquetage à manifestation virale est basé sur un rétrovirus de type C.

5. Procédé selon l'une quelconque des revendications précédentes, dans lequel on présente le premier membre de la psl comme un polypeptide de fusion.

6. Procédé selon l'une quelconque des revendications précédentes, dans lequel on présente le premier membre de la psl comme une fusion avec la partie de liaison capable d'infecter.

7. Procédé selon l'une quelconque des revendications précédentes, dans lequel on présente le premier membre de la psl comme une fusion avec une protéine à enveloppe virale.

8. Procédé selon l'une quelconque des revendications précédentes, dans lequel on présente le premier membre de la psl comme une fusion terminale en N avec une glycoprotéine à enveloppe rétrovirale.

9. Procédé selon l'une quelconque des revendications précédentes, dans lequel la partie de liaison capable d'infecter contient une glycoprotéine à enveloppe rétrovirale substantiellement intacte.

10. Procédé selon l'une quelconque des revendications précédentes, dans lequel la cellule cible est eucaryotique.

11. Procédé selon l'une quelconque des revendications précédentes, dans lequel la cellule cible est mammalienne.

12. Procédé selon l'une quelconque des revendications précédentes, dans lequel la cellule cible exprime naturellement la molécule réceptrice et le second membre de la psl.

13. Procédé selon l'une quelconque des revendications précédentes, dans lequel la cellule cible a été transfectée avec une séquence dirigeant l'expression de la molécule réceptrice et/ou du second membre de la psl.

14. Procédé selon l'une quelconque des revendications précédentes, dans lequel la pluralité des séquences d'acide nucléique codant pour des premiers membres modifiés respectifs de la psl est dérivée par l'introduction de mutations dans la séquence codant pour le premier membre non modifié de la psl.

15. Procédé selon la revendication 14, dans lequel les mutations sont introduites par un processus de mutagenèse substantiellement aléatoire.

16. Trousse pour utilisation lors de l'exécution du procédé selon l'une quelconque des revendications précédentes, comprenant une structure d'acide nucléique appropriée pour accepter une séquence codant pour un premier membre d'une psl ; des cellules d'empaquetage rétroviral pour produire des empaquetages à manifestation virale incorporant la structure d'acide nucléique ; des cellules cible exprimant sur leur surface un récepteur qui permet l'infection par les empaquetages à manifestation virale et exprimant également le second membre de la psl ; et des instructions pour exécuter un procédé conforme à l'une quelconque des revendications précédentes.

17. Trousse selon la revendication 16, comprenant en outre un ou plusieurs parmi les éléments suivants : des moyens pour introduire des mutations dans la séquence d'acide nucléique codant pour le premier membre non-modifié de la psl ; et des moyens de détection pour détecter l'infection d'une cellule cible par un empaquetage à manifestation virale.
